# EUROPEAN PATENT APPLICATION

(11) **EP 4 480 472 A1**
(43) Date of publication of application: **25.12.2024**
(21) Application number: 23755863.0
(22) Date of filing: 17.02.2023
(51) Int. Cl.: A61K 9/51, A61K 47/02, A61K 38/20, A61K 31/7088, A61K 45/06, A61K 31/05, A61P 35/00, A61P 31/12

(54) **ALUMINUM-BASED SELF-ASSEMBLED DELIVERY SYSTEM FOR MRNA, METHOD FOR PREPARING SAME, AND USE THEREOF**

(30) Priority: 18.02.2022 CN 202210151609
(71) Applicant: Guangzhou Realbenefitspot Pharmaceutical Co., Ltd., Guangzhou, Guangdong 510663 (CN)
(72) Inventor: WANG, Yaling, Guangzhou, Guangdong 510000 (CN); CHEN, Chunying, Guangzhou, Guangdong 510000 (CN); ZHAO, Yuliang, Guangzhou, Guangdong 510000 (CN)
(74) Representative: J A Kemp LLP
(86) International application number: PCT/CN2023/076665
(87) International publication number: WO 2023/155862

(57) **Abstract**

The present invention relates to the technical field of biopharmaceutics and vaccines, and in particular, to an aluminum-based self-assembled delivery system for mRNA, a method for preparing same, and use thereof. The aluminum-based self-assembled delivery system for messenger RNA is a nanocrystallite formed by in-situ self-assembly of an aluminum ion, an mRNA, and a co-assembled molecule driven by coordination. The delivery system serves as a vector-free self-delivery system serving for interleukin 21 mRNA, and can achieve efficient delivery of interleukin 21 mRNA and improved mRNA stability and targeted mRNA delivery efficiency. The nanoparticle of the present invention features good stability in serum and low toxicity, and can achieve efficient mRNA transfection in melanoma cells and significant inhibition of tumor growth in a melanoma mouse model. Therefore, the present invention provides an efficient and convenient preparation method for nucleic acid delivery systems, and has extremely high practical value.

## Description

This application claims the priority of the Chinese patent application No. 202210151609.3, filed with the China Patent Office on February 18, 2022, entitled "ALUMINUM-BASED SELF-ASSEMBLED DELIVERY SYSTEM FOR MRNA, METHOD FOR PREPARING SAME, AND USE THEREOF", which is incorporated herein by reference in its entirety.

### Technical Field

The present invention relates to the technical field of biopharmaceutics and vaccines, and in particular, to an aluminum-based self-assembled delivery system for mRNA, a method for preparing a same, and use thereof.

### Background

Cancer has now become a major disease that threatens human health. Immune checkpoint therapy significantly prolongs the progression-free survival of cancer patients, compared with traditional chemotherapy and physical therapy. However, due to the immunosuppressive microenvironment of tumors, immunotherapy induces long-lasting responses in only a minority of patients. Although rational combinations with inflammatory cytokines or immune agonists can alleviate some of the immunosuppression, systemic administration of these proteins is hampered by serious immune-related adverse events. Early phase 1 clinical trials involving some promising cytokines, such as interleukin 2 (IL-2) and interleukin 12 (IL-12), were associated with suboptimal antitumor therapeutic effects and high treatment-related morbidity and even mortality due to limited intratumoral drug exposure and overstimulation of lymphocytes in healthy tissues. Interleukin 21 (IL-21) is one of the major immunomodulators that regulates various immune responses by affecting a variety of immune cells. IL-21 can enhance the antigen-specific response of immune cells, promote the anti-tumor activity of T cells and NK cells, and plays a key role in B cell differentiation and germinal centers, and is a potential target for the development of tumor immunotherapy. However, the production of pathogenic autoantibodies can be caused by overexpression of IL-21, leading to the occurrence of autoimmune diseases. The application of IL-21 in clinical tumor treatment is ultimately limited due to severe liver or gastrointestinal toxicity issues and lack of consistent clinical activity. The development of strategies to efficiently deliver cytokines to tumor sites is of utmost importance. Anti-tumor responses can be achieved by gene therapy based on the delivery of IL-21 without inducing vascular leakage syndrome.

The anti-tumor function of IL-21 by plasmid DNA transfection has been confirmed (Cancer Res. 2003, 63, 9016.), however, the delivery of plasmid DNA is limited by the instability of in vivo delivery, and hampered by the reliance on nuclear localization and the risk of gene integration. mRNA can also provide rapid protein expression without relying on nuclear localization and without the risk of gene integration, making it a better choice for intratumoral delivery of IL-21. However, the delivery of mRNA also has several potential challenges, including its large size, high negative charge, and susceptibility to degradation, which can lead to suboptimal translation of a target protein if it is not efficiently modified and delivered into cells. Therefore, it remains challenging to safely and effectively deliver mRNA to tumor cells in vivo while maintaining integrity and functional activity.

### Summary of the Invention

In view of this, the present invention provides an aluminum-based self-assembled delivery system for mRNA, a method for preparing a same, and use thereof. The aluminum-based self-assembled delivery system for mRNA is a nanocrystallite formed by in-situ self-assembly of an aluminum ion, an mRNA of cytokines such as interleukin 21, and a co-assembled molecule driven by coordination. The delivery system serves as a vector-free self-delivery system serving for interleukin 21 mRNA, and can achieve efficient delivery of interleukin 21 mRNA and improved mRNA stability and targeted mRNA delivery efficiency. The nanoparticle of the present invention features good stability in serum and low toxicity, and can achieve efficient mRNA transfection in melanoma cells and significant inhibition of tumor growth when topically administered in a melanoma mouse model.

In order to achieve the above-mentioned object of the invention, the present invention provides the following technical solutions:
The present invention provides an aluminum-based self-assembled delivery system for mRNA, wherein the delivery system is formed by self-assembly of an aluminum ion, an mRNA and a co-assembled molecule through coordination interaction.

Preferably, the mRNA is a single-stranded ribonucleotide carrying the genetic information of cytokines, and further preferably is the mRNA of interleukin 21.

Preferably, the co-assembled molecule is a polyhydroxy small-drug-molecule, the polyhydroxy small-drug-molecule is selected from at least one of resveratrol, rhein, tannic acid, gallic acid, tea polyphenol, and dopamine.

In a second aspect, the present invention provides a method for preparing the aluminum-based self-assembled delivery system as described in the first aspect, the preparation method comprising the following steps:
(1) adding an mRNA and a co-assembled molecule into a solvent, and mixing by stirring or vortexing to obtain a mixed solution 1;
(2) mixing an aluminum salt solution and the mixed solution 1 evenly by stirring or vortexing to obtain a mixed solution 2; and
(3) adding an alkaline solution to the mixed solution 2 to adjust the pH, and reacting them at a constant temperature, and after cooling, centrifugation-washing the product to obtain the aluminum-based self-assembled delivery system for mRNA.

In the step (1), the temperature of the stirring is 4-25°C, the time of the stirring is 1-20 min, and the rate of the stirring is 100-600 rpm.

In the step (3), the time of the constant temperature reaction is 0.5 to 24 h. Preferably, the rotation speed of the centrifugation-washing is 6000-18407 g/min, the time of the centrifugation is 1-15 min/time, and the number of the centrifugation is 1-5 times.

Preferably, the concentration of the aluminum ion in the aluminum salt solution is 1-100 mM.

Preferably, the concentration of the mRNA in the mixed solution 1 is 0.005-1.0 mM.

Preferably, the concentration of the co-assembled molecule is 1-100 mM.

Preferably, the molar ratio of the aluminum salt to the co-assembled molecule in the mixed solution 2 is (100:1) to (1:100).

Preferably, the molar ratio of the aluminum ion to the mRNA in the mixed solution 2 is (1-500):1.

Preferably, the alkaline solution is one or more of NaOH solution, KOH solution, sodium phosphate solution, sodium pyrophosphate solution, sodium carbonate solution, sodium bicarbonate solution, and ammonia water; and the concentration of the alkaline solution is 5-1000 mM.

Preferably, in the step (2), the speed of the stirring is 0-1400 rpm, and the time of the stirring is 5-300 min.

Preferably, in the step (2), the speed of the stirring is 100-800 rpm.

Preferably, in the step (3), the speed of the constant temperature reaction is 100-1400 rpm and the time is 30-360 min.

Preferably, in the step (3), the pH is 6.0-10.0; and the temperature of the constant temperature reaction is 25-90°C.

In a third aspect, the present invention provides the use of the aluminum-based self-assembled delivery system for mRNA in the preparation of a drug delivery system.

In a fourth aspect, the present invention provides the use of the aluminum-based self-assembled delivery system for mRNA in the preparation of an anti-tumor or anti-viral drug delivery system.

Preferably, the use includes any one or any combination of intratumor injection, intramuscular injection, subcutaneous injection, intradermal injection, intravenous injection, and application to mucosa.

In a fifth aspect, the present invention provides a drug delivery system, comprising the aluminum-based self-assembled delivery system for mRNA or the aluminum-based self-assembled delivery system for mRNA prepared by the preparation method, and a drug.

In a sixth aspect, the present invention provides a method for preventing or treating tumors or viruses, comprising administering the aluminum-based self-assembled delivery system for mRNA, the aluminum-based self-assembled delivery system for mRNA prepared by the preparation method, or the drug delivery system.

The present invention has the following technical effects:
The aluminum-based self-assembly provided by the present invention can effectively bind to mRNA, and can effectively increase the level of mRNA entering cells, improve the serum stability of mRNA, and reduce systemic toxicity. The preparation method is scientific and efficient, has low requirements on the level of technical personnel, and has broad application prospects.

Compared with the existing mRNA delivery technology, the present invention has the following beneficial effects:
(1) the co-assembled molecule (anti-tumor and antiviral drug), messenger RNA and aluminum ion of the present invention are assembled inside the same nanoparticle, such that they can be delivered into cells at the same time, thus having a synergistic therapeutic effect; and
(2) the co-assembled particle contains metal ions to stabilize the nucleic acid structure, improving the stability of the particles in serum, and reducing the risk of toxicity caused by vascular leakage.

### Description of the Drawings

FIG. 1 shows a TEM image of the aluminum-based self-assembled particle obtained in Example 1 of the present invention;
FIG. 2 shows a TEM image of the aluminum-based self-assembled particle obtained in Example 6 of the present invention;
FIG. 3 shows a TEM image of the aluminum-based self-assembled particle obtained in Example 9 of the present invention;
FIG. 4 shows the cytotoxicity results of the aluminum-based self-assembled particle mRRAl-001 obtained in Example 1 of the present invention;
FIG. 5 shows the cytotoxicity results of the aluminum-based self-assembled particles obtained in Examples 1-17 of the present invention;
FIG. 6 shows the intracellular mRNA delivery results of mRRAl-006C after the aluminum-based self-assembled particle obtained in Example 6 of the present invention is labeled with Cy5 fluorescent dye;
FIG. 7 shows the weight of mice after treating tumor model mice with the aluminum-based self-assembled particle mRRAl-001 obtained in Example 1 of the present invention, in Example 21 of the present invention;
FIG. 8 shows the tumor volume after treating tumor model mice with the aluminum-based self-assembled particle mRRAl-001 obtained in Example 1 of the present invention, in Example 21 of the present invention;
FIG. 9 shows the survival rate after treating tumor model mice with the aluminum-based self-assembled particle mRRAl-001 obtained in Example 1 of the present invention, in Example 21 of the present invention;
FIG. 10 shows the tumor suppression after treating tumor model mice with the aluminum-based self-assembled particles obtained in other representative examples of the present invention, in Example 21 of the present invention;
FIG. 11 shows the results of detecting the intratumoral key factor IL-21+cells after treating tumor model mice in the treatment group with the aluminum-based self-assembled particle mRRAl-001 obtained in Example 1 of the present invention, the treatment group with free mRNA and the blank control group, in Example 22 of the present invention;
FIG. 12 shows the results of detecting the intratumoral key factor interferon IFN-γ expression positive cells after treating tumor model mice in the treatment group with the aluminum-based self-assembled particle mRRAl-001 obtained in Example 1 of the present invention, the treatment group with free mRNA and the blank control group, in Example 22 of the present invention;
FIG. 13 shows the results of detecting the percentage of the intratumoral key factor CD8+ T cells after treating tumor model mice in the treatment group with the aluminum-based self-assembled particle mRRAl-001 obtained in Example 1 of the present invention, the treatment group with free mRNA and the blank control group, in Example 22 of the present invention; and

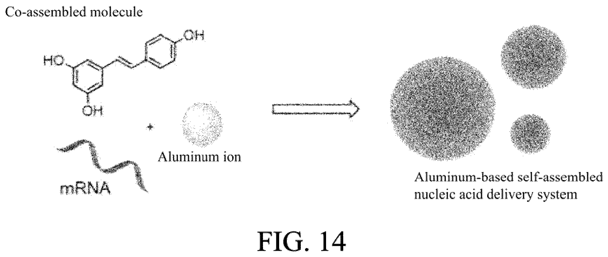

FIG. 14 shows a schematic diagram showing a method for preparing the aluminum-based self-assembled delivery system of the present invention.

### Detailed Description

The present invention discloses an aluminum-based self-assembled delivery system for mRNA, a method for preparing a same, and use thereof, which can be implemented by those skilled in the art with reference to the contents herein with appropriate improvements to process parameters. It should be particularly noted that all similar substitutions and alterations are obvious to those skilled in the art and are deemed to be included in the present invention. The method and use of the present invention have been described through preferred examples. It is obvious that related persons can make alterations or appropriate changes and combinations of the method and use described herein without departing from the content, spirit, and scope of the present invention, to implement and apply the technology of the present invention.

The raw materials and reagents used in the method for preparing the aluminum-based self-assembled delivery system for interleukin 21 messenger RNA (mRNA) provided by the present invention, and in the anti-tumor use can all be purchased from the market. Among them, the main raw materials include: resveratrol, purchased from McLean, CAS: 501-36-0; IL-21 mRNA.

The present invention will be further described below in conjunction with the examples:

### Example 1

360 µL of an aqueous solution containing 5 nmol of mRNA was mixed with 20 µL of resveratrol (20 mM) by vortexing at 23°C for 1 minute to obtain a mixed solution 1, and subsequently 20 µL of aqueous AlCl₃·6H₂O (20 mM) solution was added to the mixed solution 1 and then vortexed for 10 seconds to obtain a mixed solution 2. Then, the pH was adjusted to about 7 with 100 mM NaOH, and the mixture was heated in a 95°C metal bath for 3 hours. The product was centrifugation-washed 3 times at 13,000 g/min to obtain mRNA-resveratrol-aluminum self-assembled nanoparticle (mRRAl-001).

### Example 2

360 µL of an aqueous solution containing 5 nmol of mRNA was mixed with 20 µL of resveratrol (20 mM) by vortexing at 15°C for 1 minute to obtain a mixed solution 1, and subsequently 20 µL of aqueous Al(NO₃)₃·6H₂O (20 mM) solution was added to the mixed solution 1 and then stirred at 100 rpm for 240 minutes to obtain a mixed solution 2. Then, the pH was adjusted to about 7 with 100 mM sodium phosphate, and the mixture was stirred at 100 rpm for 1 hour at 50°C. The product was centrifugation-washed 3 times at 13,000 g/min to obtain mRNA-resveratrol-aluminum self-assembled nanoparticle (mRRAl-002).

### Example 3

320 µL of an aqueous solution containing 5 nmol of mRNA was mixed with 40 µL of resveratrol (20 mM) by vortexing at 16°C for 1 minute to obtain a mixed solution 1, and subsequently 40 µL of aqueous AlCl₃·6H₂O (20 mM) solution was added to the mixed solution 1 and then stirred at 100 rpm for 180 minutes to obtain a mixed solution 2. Then, the pH was adjusted to about 6.5 with 100 mM KOH, and the mixture was stirred at 100 rpm for 3 hours at 75°C. The product was centrifugation-washed 3 times at 13,000 g/min to obtain mRNA-resveratrol-aluminum self-assembled nanoparticle (mRRAl-003).

### Example 4

320 µL of an aqueous solution containing 5 nmol of mRNA was mixed with 40 µL of resveratrol (20 mM) by vortexing at 25°C for 1 minute to obtain a mixed solution 1, and subsequently 40 µL of aqueous Al₂(SO₄)₃·6H₂O (20 mM) solution was added to the mixed solution 1 and then stirred at 100 rpm for 90 minutes to obtain a mixed solution 2. Then, the pH was adjusted to about 6.5 with 100 mM sodium pyrophosphate, and the mixture was stirred at 100 rpm for 6 hours at 50°C. The product was centrifugation-washed 3 times at 13,000 g/min to obtain mRNA-resveratrol-aluminum self-assembled nanoparticle (mRRAl-004).

### Example 5

320 µL of an aqueous solution containing 5 nmol of mRNA was mixed with 40 µL of resveratrol (80 mM) by vortexing at 24°C for 1 minute to obtain a mixed solution 1, and subsequently 40 µL of aqueous Al (NO₃)₃·6H₂O (40 mM) solution was added to the mixed solution 1 and then stirred at 100 rpm for 80 minutes to obtain a mixed solution 2. Then, the pH was adjusted to about 7.5 with 100 mM sodium carbonate, and the mixture was stirred at 100 rpm for 6 hours at 30°C. The product was centrifugation-washed 3 times at 13,000 g/min to obtain mRNA-resveratrol-aluminum self-assembled nanoparticle (mRRAl-005).

### Example 6

320 µL of an aqueous solution containing 5 nmol of mRNA-Cy5 was mixed with 40 µL of resveratrol (40 mM) by vortexing at 19°C for 1 minute to obtain a mixed solution 1, and subsequently 40 µL of aqueous AlAc₃·6H₂O (80 mM) solution was added to the mixed solution 1 and then stirred at 100 rpm for 60 minutes to obtain a mixed solution 2. Then, the pH was adjusted to about 8.0 with 100 mM NaOH, and the mixture was stirred at 100 rpm for 3 hours at 60°C. The product was centrifugation-washed 3 times at 13,000 g/min to obtain mRNA-resveratrol-aluminum self-assembled nanoparticle (mRRAl-006).

### Example 7

360 µL of an aqueous solution containing 5 nmol of mRNA was mixed with 20 µL of tannic acid (40 mM) by vortexing at 17°C for 1 minute to obtain a mixed solution 1, and subsequently 20 µL of aqueous AlAc₃·6H₂O (100 mM) solution was added to the mixed solution 1 and then stirred at 100 rpm for 40 minutes to obtain a mixed solution 2. Then, the pH was adjusted to about 9.5 with 100 mM KOH, and the mixture was stirred at 100 rpm for 3 hours at 25°C. The product was centrifugation-washed 3 times at 13,000 g/min to obtain mRNA-tannic acid-aluminum self-assembled nanoparticle (mRRAl-007).

### Example 8

360 µL of an aqueous solution containing 5 nmol of mRNA was mixed with 20 µL of gallic acid (60 mM) by vortexing at 24°C for 1 minute to obtain a mixed solution 1, and subsequently 20 µL of aqueous AlCl₃·6H₂O (100 mM) solution was added to the mixed solution 1 and then stirred at 100 rpm for 30 minutes to obtain a mixed solution 2. Then, the pH was adjusted to about 7.5 with 100 mM ammonia water, and the mixture was stirred at 100 rpm for 3 hours at 50°C. The product was centrifugation-washed 3 times at 13,000 g/min to obtain mRNA-gallic acid-aluminum self-assembled nanoparticle (mRRAl-008).

### Example 9

360 µL of an aqueous solution containing 5 nmol of mRNA was mixed with 20 µL of dopamine (100 mM) by vortexing at 14°C for 1 minute to obtain a mixed solution 1, and subsequently 20 µL of aqueous AlCl₃·6H₂O (100 mM) solution was added to the mixed solution 1 and then stirred at 100 rpm for 150 minutes to obtain a mixed solution 2. Then, the pH was adjusted to about 6.5 with 100 mM NaOH, and the mixture was stirred at 100 rpm for 3 hours at 50°C. The product was centrifugation-washed 3 times at 13,000 g/min to obtain mRNA-dopamine-aluminum self-assembled nanoparticle (mRRAl-009).

### Example 10

360 µL of an aqueous solution containing 5 nmol of mRNA and 20 µL of resveratrol (100 mM) were stirred at 100 rpm for 20 minutes at 25°C to obtain a mixed solution 1, and subsequently 20 µL of aqueous Al(NO₃)₃·6H₂O (1 mM) solution was added to the mixed solution 1 and then stirred at 100 rpm for 5 minutes to obtain a mixed solution 2. Then, the pH was adjusted to about 7.5 with 5 mM sodium phosphate, and the mixture was stirred at 100 rpm for 1 hour at 50°C. The product was centrifugation-washed 3 times at 15,000 g/min to obtain mRNA-resveratrol-aluminum self-assembled nanoparticle (mRRAl-010).

### Example 11

320 µL of an aqueous solution containing 0.32 µmol of mRNA was mixed with 40 µL of rhein (1 mM) by stirring at 200 rpm for 10 minutes at 14°C to obtain a mixed solution 1, and subsequently 40 µL of aqueous AlCl₃·6H₂O (100 mM) solution was added to the mixed solution 1 and then stirred at 100 rpm for 300 minutes to obtain a mixed solution 2. Then, the pH was adjusted to about 8.5 with 100 mM KOH, and the mixture was stirred at 100 rpm for 3 hours at 75°C. The product was centrifugation-washed 3 times at 12,000 g/min to obtain mRNA-rhein-aluminum self-assembled nanoparticle (mRRAl-011).

### Example 12

400 µL of an aqueous solution containing 200 nmol of mRNA was mixed with 30 µL of gallic acid (100 mM) by stirring at 300 rpm for 8 minutes at 20°C to obtain a mixed solution 1, and subsequently 30 µL of aqueous Al₂(SO₄)₃·6H₂O (50 mM) solution was added to the mixed solution 1 and then stirred at 800 rpm for 100 minutes to obtain a mixed solution 2. Then, the pH was adjusted to about 6.5 with 50 mM sodium pyrophosphate, and the mixture was stirred at 100 rpm for 6 hours at 90°C. The product was centrifugation-washed twice at 9,000 g/min to obtain mRNA-gallic acid-aluminum self-assembled nanoparticle (mRRAl-012).

### Example 13

120 µL of an aqueous solution containing 150 nmol of mRNA was mixed with 20 µL of tea polyphenol (10 mM) by stirring at 400 rpm for 6 minutes at 8°C to obtain a mixed solution 1, and subsequently 20 µL of aqueous Al(NO₃)₃·6H₂O (80 mM) solution was added to the mixed solution 1 and then stirred at 300 rpm for 250 minutes to obtain a mixed solution 2. Then, the pH was adjusted to about 7.5 with 1000 mM sodium carbonate, and the mixture was stirred at 1400 rpm for 30 minutes at 30°C. The product was centrifugation-washed 4 times at 16,000 g/min to obtain mRNA-tea polyphenol-aluminum self-assembled nanoparticle (mRRAl-013).

### Example 14

180 µL of an aqueous solution containing 50 nmol of mRNA-Cy5 was mixed with 50 µL of dopamine (40 mM) by stirring at 500 rpm for 4 minutes at 12°C to obtain a mixed solution 1, and subsequently 50 µL of aqueous AlAc₃·6H₂O (80 mM) solution was added to the mixed solution 1 and then stirred at 500 rpm for 50 minutes to obtain a mixed solution 2. Then, the pH was adjusted to about 8.0 with 100 mM NaOH, and the mixture was stirred at 700 rpm for 3 hours at 60°C. The product was centrifugation-washed twice at 18,407 g/min to obtain mRNA-dopamine-aluminum self-assembled nanoparticle (mRRAl-014).

### Example 15

300 µL of an aqueous solution containing 20 nmol of mRNA was mixed with 20 µL of tannic acid (40 mM) by stirring at 600 rpm for 2 minutes at 18°C to obtain a mixed solution 1, and subsequently 20 µL of aqueous AlAc₃·6H₂O (100 mM) solution was added to the mixed solution 1 and then stirred at 700 rpm for 200 minutes to obtain a mixed solution 2. Then, the pH was adjusted to about 9.5 with 100 mM KOH, and the mixture was stirred at 200 rpm for 2 hours at 25°C. The product was centrifugation-washed 5 times at 10,000 g/min to obtain mRNA-tannic acid-aluminum self-assembled nanoparticle (mRRAl-015).

### Example 16

360 µL of an aqueous solution containing 5 nmol of mRNA was mixed with 20 µL of gallic acid (60 mM) by vortexing at 4°C for 1 minute to obtain a mixed solution 1, and subsequently 20 µL of aqueous AlCl₃·6H₂O (100 mM) solution was added to the mixed solution 1 and then stirred at 100 rpm for 5 minutes to obtain a mixed solution 2. Then, the pH was adjusted to about 7.5 with 200 mM ammonia water, and the mixture was stirred at 100 rpm for 1 hour at 50°C. The product was centrifugation-washed 3 times at 8,000 g/min to obtain mRNA-gallic acid-aluminum self-assembled nanoparticle (mRRAl-016).

### Example 17

360 µL of an aqueous solution containing 5 nmol of mRNA was mixed with 20 µL of dopamine (100 mM) by vortexing at 10°C for 1 minute to obtain a mixed solution 1, and subsequently 20 µL of aqueous AlCl₃·6H₂O (100 mM) solution was added to the mixed solution 1 and then stirred at 300 rpm for 20 minutes to obtain a mixed solution 2. Then, the pH was adjusted to about 6.5 with 100 mM NaOH, and the mixture was stirred at 900 rpm for 30 minutes at 50°C. The product was centrifugation-washed once at 6,000 g/min to obtain mRNA-dopamine-aluminum self-assembled nanoparticle (mRRAl-017).

### Example 18

### (1) Characterization of morphology for aluminum-based self-assembled delivery system

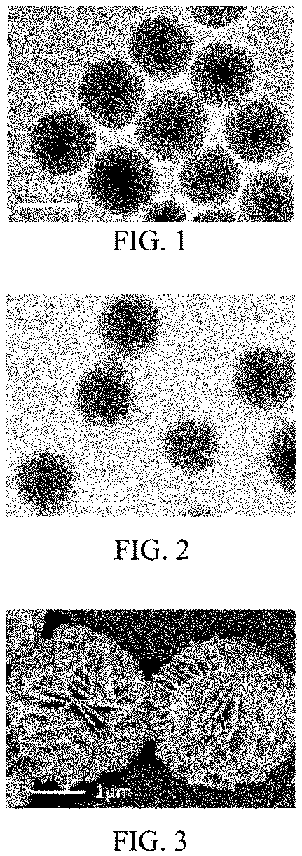

At 25°C, the aluminum-based self-assembled particles represented by Examples 1, 6, and 9 were diluted to a concentration of 10 µg/mL with ultrapure water and dropped onto a common carbon support film. After natural drying, the structure of the aluminum-based self-assembly was observed under an electron microscope, and the structure appeared to be granular under a transmission electron microscope (FEI Company, model Tecnai G2 20S-TWIN). The results are shown in Figs. 1-3, where Fig. 1 is an electron microscope image of the product of Example 1, Fig. 2 is an electron microscope image of the product of Example 6, and Fig. 3 is an electron microscope image of the product of Example 9. Figs. 1-3 show that the self-assembly of metal-polyphenol complexes can result in self-assembled particles of uniform size, based on a variety of reaction conditions with different ratios and concentrations.

### (2) Characterization of the hydration particle size for the aluminum-based self-assembly

At 25°C, the concentration of the aluminum-based self-assembled particles obtained in Examples 1-17 was diluted to 10 µg/mL, and the particle size of the aluminum-based self-assembly was measured using a nanoparticle size analyzer (purchased from Malvern, Zetasizer Nano ZS model). The results are shown in Table 1.

The physical and chemical properties of the aluminum-based self-assembled particles prepared in Examples 1-17 are shown in Table 1.

**Table 1**

| Sample | Hydration particle size | Sample | Hydration particle size |
|---|---|---|---|
| Example 1 | 130 nm | Example 10 | 379 nm |
| Example 2 | 140 nm | Example 11 | 268 nm |
| Example 3 | 340 nm | Example 12 | 547 nm |
| Example 4 | 670 nm | Example 13 | 836 nm |
| Example 5 | 2.27 µm | Example 14 | 846 nm |
| Example 6 | 135 nm | Example 15 | 1071 nm |
| Example 7 | 1.92 µm | Example 16 | 327 nm |
| Example 8 | 2.89 µm | Example 17 | 478 nm |
| Example 9 | 3.50 µm | | |

As shown in Table 1, all the examples of the present invention can result in nano-sized aluminum-based self-assembled nanoparticles.

### Example 19

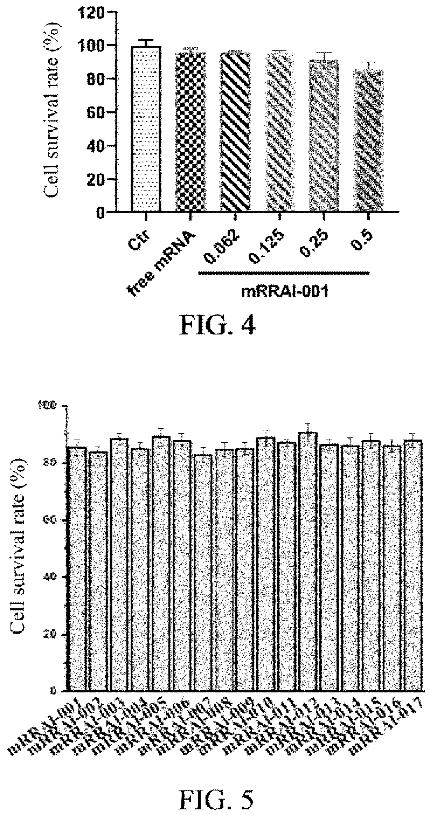

This example is mainly to evaluate the in vitro cytotoxicity of the self-assembled nanoparticles prepared in Example 1- Example 17. Cells were seeded in a suitable number on a 96-well plate and allowed to attach and grow until the density was about 80%. Cells were transfected with mRRAl-001 constructed in Example 1 at different mRNA concentrations (0.062, 0.125, 0.250 and 0.500 µg mL⁻¹) for 16 hours, and the culture supernatant was removed. After washing the cells once with PBS, 10% CCK-8 working solution was added, and the cells were incubated for another 2 h. The detection solution was then transferred into a new 96-well cell culture plate, and the absorbance values at 450 nm and 600 nm were detected and compared. The results are shown in FIG. 4. As can be seen from FIG. 4, 85.5% of the B16F10 cells still survived at the highest IL-21 mRNA concentration of 0.5 µg mL⁻¹, indicating that the mRRAl-001 self-assembled nanoparticles constructed in Example 1 of the present invention have very low cytotoxicity.

At the same time, similar cytotoxicity evaluation was performed using mRRAl-002 to mRRAl-017 constructed in Examples 2-17 with the highest mRNA concentration of 0.500 µg mL⁻¹, and the results are shown in Fig. 5. As can be seen from Fig. 5, the self-assembled nanoparticles constructed in Examples 1-17 all had a B16F10 cell survival rate higher than 83% at the highest IL-21 mRNA concentration of 0.5 µg mL⁻¹, indicating that the self-assembled nanoparticles constructed in Examples 1-17 of the present invention all have very low cytotoxicity.

### Example 20

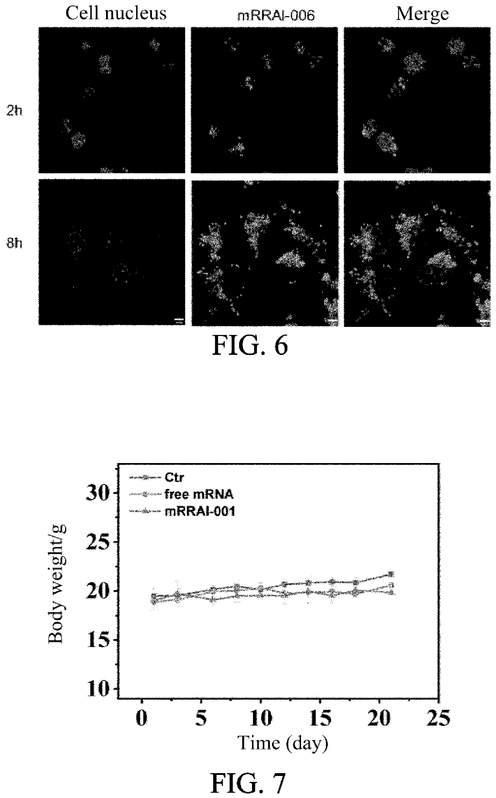

This example evaluates the ability of the self-assembled particle represented by mRRAl prepared in Example 6 of the present invention to deliver interleukin 21-mRNA into cells. In this example, IL-21 mRNA was labeled with Cy5 and the self-assembled nanoparticle mRRAl-006C was prepared based on the same preparation conditions as in Example 6. B16F10 cells were seeded into a glass-bottomed dish and treated with mRRAl-006 for 2 h. After 8 h, the cells were fixed, stained for the cell nuclei with Hoechst33342, and observed under a laser confocal microscope. The results are shown in Fig. 6. The fluorescence of the cell nucleus (labeled with Hoechst33342) and mRRAl-006C (labeled with Cy5) is illustrated by the laser confocal images in Fig. 6. The amount of intracellular mRRAl-006C gradually increased with the incubation time (Fig. 6), which can be attributed to the time-dependent increase in internalization of mRRAl-006C in cells.

### Example 21

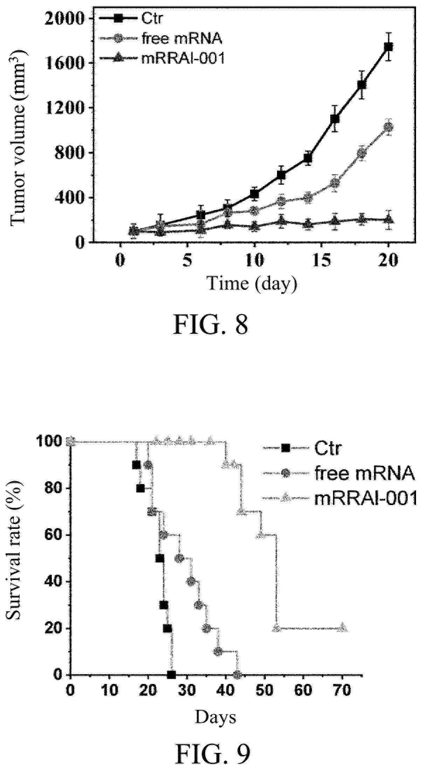

BALB/c nude mice (6-7 weeks) were inoculated with B16F10 melanoma at the back of the right leg. When the tumor volume reached ~100 mm³, the mice were randomly divided into 3 groups according to body weight, namely: a blank control group (Ctrl), a control group with free mRNA (1 nmol), and a group with the self-assembled nanoparticle mRRAl-001 (containing 1 nmol mRNA) represented by Example 1 of the present application. The material and drug were injected intratumorally, and the day was recorded as day 0. The mice in each group were treated accordingly on day 1, day 4, and day 7. The tumor volumes of mice in each group were recorded every two days. The tumor volume (V, mm³) of each mouse was calculated using the following equation: Volume V = length × width²/2; the results are shown in Figs. 4-8, where Fig. 7 shows the weight changes of mice in each group, Fig. 8 shows the tumor volume changes of mice in each group, and Fig. 9 shows the survival rate of mice in each group.

As can be seen from Fig. 7, the weight changes of mice in each group are not great; as can be seen from Fig. 8, the average tumor size of the control group mice is 1746 mm³ at 20 days; the growth of tumors can be inhibited to a certain extent in the free mRNA group, with an average tumor size of 1028 mm³, while the growth of tumors can be effectively inhibited using mRRAl-001 of the present application, with an average tumor size of 201.7 mm³; and as can be seen from Fig. 9, the longest survival time of the control mice is 26 days, the longest survival time of the free mRNA mice is 43 days, and some mice in the mRRAl-001 group are still alive at 70 days, and the survival time of the remaining mice in the group is also significantly extended. The results demonstrated that the use of mRRAl-001 can significantly inhibit tumor growth and increase the survival rate and prolong the survival time of mice. mRRAl-001 can be used as a tumor immunopotentiator to enhance the anti-tumor in situ effect in the body.

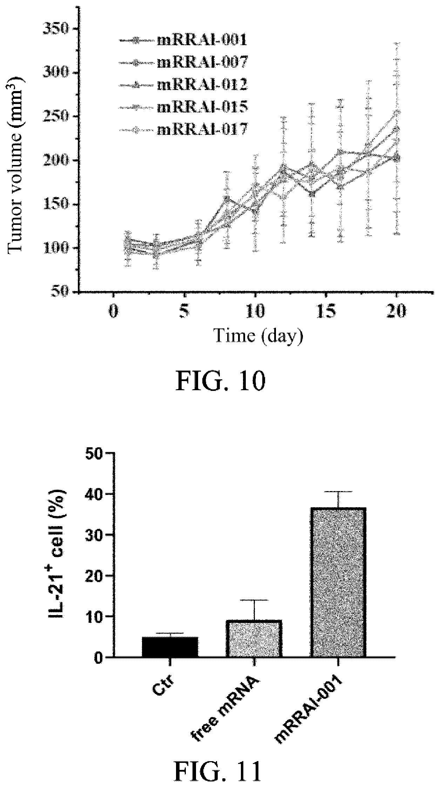

At the same time, the present invention uses similar conditions to evaluate the anti-tumor in situ effect of representative nanoparticles of other examples, such as mRRAl-007, mRRAl-012, mRRAl-015, and mRRAl-017 constructed in Examples 7, 12, 15, and 17, and the results are shown in Fig. 10. It can be seen from Fig. 10 that for the self-assembled nanoparticles constructed, the average tumor size of mice in each experimental group of assembled nanoparticles ranges from 190 to 260 mm³ at 20 days, suggesting that the self-assembled nanoparticles constructed can effectively inhibit tumor growth.

### Example 22

Tumor inhibition ability is the most direct indicator for verifying delivery efficiency. The several particles obtained in the examples of the present invention can significantly inhibit the growth of melanoma, and their mechanisms of action are basically similar; therefore, only the experimental group treated with mRRAl-001 was selected for subsequent verification of mechanisms such as intracellular interferon expression.

After the mice investigated in Example 21 were euthanized, their tumors were dissected and digested with collagenase IV and DNase at 37°C, and then filtered to prepare single-cell suspensions. Each sample was stained with fluorescently conjugated IL-21, INF-γ, and CD8+ T cell-specific antibodies at 4°C and then fixed in 4% PFA and analyzed by flow cytometry. Data were analyzed using FlowJo V10 software. The experimental results are shown in Figs. 11-13.

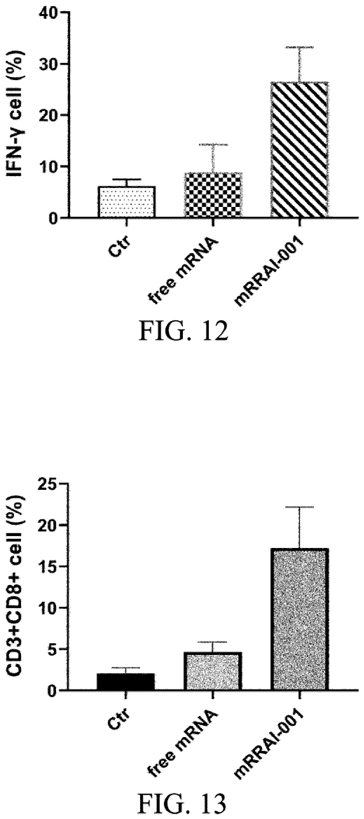

As shown in Figs. 11-13, 36.8% IL-21⁺cells are present in the tumor tissues treated with mRRAl-001 after different drug treatments, which is about 4 times that of the tumors in the free mRNA treatment group (Fig. 11), verifying the effective transfection efficiency of mRRAl-001 in tumors. 26.45% interferon IFN-γ-expression positive cells are present in the tumors treated with mRRAl-001, which is 3 times that of the free mRNA treatment group (Fig. 12), while the percentage of CD8+ T cells was 17.2%, which is 3.7 times that of the free group (Fig. 13). The results in Figs. 11-13 fully demonstrate that mRRAl-001 is a very effective delivery system for interleukin 21 mRNA.

The above is a detailed introduction to the aluminum-based self-assembled delivery system for mRNA, a method for preparing a same, and use thereof provided by the present invention. Specific examples are used herein to illustrate the principles and embodiments of the present invention. The description of the above examples is only used to help understand the method and core concept of the present invention It should be noted that for those skilled in the art, several improvements and modifications may be made to the present invention without departing from the principle of the present invention, and these improvements and modifications also fall within the protection scope of the claims of the present invention.

## Claims

1. An aluminum-based self-assembled delivery system for mRNA, wherein the delivery system is formed by self-assembly of an aluminum ion, an mRNA and a co-assembled molecule through coordination interaction.

2. The aluminum-based self-assembled delivery system for mRNA according to claim 1, wherein the mRNA is a single-stranded ribonucleotide carrying the genetic information of cytokines.

3. The aluminum-based self-assembled delivery system for mRNA according to claim 2, wherein the mRNA is cytokine mRNA.

4. The aluminum-based self-assembled delivery system for mRNA according to claim 3, wherein the mRNA is interleukin 21 mRNA.

5. The aluminum-based self-assembled delivery system according to claim 1, wherein the co-assembled molecule is a polyhydroxy small-drug-molecule;
the polyhydroxy small-drug-molecule comprises at least one of resveratrol, rhein, tannic acid, gallic acid, tea polyphenol and dopamine.

6. A method for preparing the aluminum-based self-assembled delivery system for mRNA according to any one of claims 1-5, comprising the following steps:
(1) adding an mRNA and a co-assembled molecule into a solvent, and mixing by stirring or vortexing to obtain a mixed solution 1;
(2) mixing an aluminum salt solution and the mixed solution 1 evenly by stirring or vortexing to obtain a mixed solution 2; and
(3) adding an alkaline solution to the mixed solution 2 to adjust the pH, and reacting them at a constant temperature, and after cooling, centrifugation-washing the product to obtain the aluminum-based self-assembled delivery system for mRNA.

7. The preparation method according to claim 6, wherein in the step (1), the temperature of the stirring is 4-25°C, the time of the stirring is 1-20 min, and the rate of the stirring is 100-600 rpm.

8. The preparation method according to claim 6, wherein in the step (3), the time of the constant temperature reaction is 0.5-24 h, the speed of the centrifugation-washing is 6000-18407 g/min, the time of the centrifugation is 1-15 min/time, and the number of the centrifugation is 1-5 times.

9. The preparation method according to claim 6, wherein the concentration of the aluminum ions in the aluminum salt solution is 1-100 mM.

10. The preparation method according to claim 6, wherein the concentration of the mRNA in the mixed solution 1 is 0.005-1.0 mM.

11. The preparation method according to claim 6, wherein the concentration of the co-assembled molecule is 1-100 mM.

12. The preparation method according to claim 6, wherein the molar ratio of the aluminum ions in the aluminum salt solution to the co-assembled molecule is (100: 1) to (1:100).

13. The preparation method according to claim 6, wherein the molar ratio of the aluminum ions in the aluminum salt solution to the mRNA is (1-500): 1.

14. The preparation method according to claim 6, wherein the alkaline solution is one or more of NaOH solution, KOH solution, sodium phosphate solution, sodium pyrophosphate solution, sodium carbonate solution, sodium bicarbonate solution, and ammonia water; and the concentration of the alkaline solution is 5-1000 mM.

15. The preparation method according to claim 6, wherein in the step (2), the speed of the stirring is 0-1400 rpm, and the time of the stirring is 5-300 min.

16. The preparation method according to claim 6, wherein in the step (2), the speed of the stirring is 100-800 rpm.

17. The preparation method according to claim 6, wherein in the step (3), the speed of the constant temperature reaction is 100-1400 rpm and the time is 30 min-360 min.

18. The preparation method according to claim 6, wherein in the step (3), the pH is 6.0-10.0; and the temperature of the constant temperature reaction is 25-90°C.

19. Use of the aluminum-based self-assembled delivery system for mRNA according to any one of claims 1-5 or the aluminum-based self-assembled delivery system for mRNA prepared by the preparation method according to any one of claims 6-18 in the preparation of a drug delivery system.

20. Use of the aluminum-based self-assembled delivery system for mRNA according to any one of claims 1-5 or the aluminum-based self-assembled delivery system for mRNA prepared by the preparation method according to any one of claims 6-18 in the preparation of an anti-tumor or anti-viral drug delivery system.

21. The use according to claim 19 or 20, wherein the use includes any one or any combination of intratumor injection, intramuscular injection, subcutaneous injection, intradermal injection, intravenous injection, and application to mucosa.

22. A drug delivery system, comprising the aluminum-based self-assembled delivery system for mRNA according to any one of claims 1-5 or the aluminum-based self-assembled delivery system for mRNA prepared by the preparation method according to any one of claims 6-18, and a drug.

23. A method for preventing or treating tumors or viruses, comprising administrating the aluminum-based self-assembled delivery system for mRNA according to any one of claims 1-5, the aluminum-based self-assembled delivery system for mRNA prepared by the preparation method according to any one of claims 6-18, or the drug delivery system according to claim 22.
